# FASCICULE DE BREVET EUROPEEN

(11) **EP 1 864 688 B1**
(45) Date de publication et mention de la délivrance du brevet: **16.09.2009**
(21) Numéro de dépôt: 07447034.5
(22) Date de dépôt: 04.06.2007
(51) Int. Cl.: A61L 29/12

(54) **Bouchon pour l'obturation d'une embase creuse de cathéter**
Stopfen zum Abdichten eines hohlen Kathetersockels
Stopper for blocking a hollow catheter inlet

(30) Priorité: 08.06.2006 BE 200600315
(43) Date de publication de la demande: 12.12.2007
(73) Titulaire: Belgian Diagnostic Company S.A., 6900 Marche-en-Famenne (BE)
(72) Inventeur: Ariah, Adil, 4607 Bombaye (Dalhem) (BE); De Gryse, Michel, 1653 Dworp (BE)
(74) Mandataire: Cauchie, Daniel

(56) Documents cités:
- EP-A1- 0 845 279
- WO-A-00/12171
- WO-A-99/31178
- WO-A-99/52578
- GB-A- 2 088 215
- US-A1- 2004 242 795

## Description

La présente invention se rapporte, d'une manière générale, à un bouchon pour l'obturation d'une embase creuse de cathéter.

Plus précisément, l'invention concerne un bouchon, réalisé en une composition élastomérique, pour l'obturation d'une embase creuse de cathéter reliée à une canule, en particulier une embase de cathéter conformé en Y.

On a décrit dans le brevet GB 2 088 215 un cathéter dont l'embase creuse ou chambre de raccord avec la canule comprend un élément d'obturation de celle-ci sous la forme d'un bouchon en caoutchouc siliconé. Ce bouchon comporte une extrémité distale se terminant de manière adjacente à la jonction entre l'embase et une branche latérale qui forme un angle avec cette embase, conférant à l'ensemble la forme d'un Y.

L'utilisation de caoutchouc siliconé pour la réalisation d'un tel système de bouchage a révélé toutefois certains inconvénients.

En effet, lors du retrait de l'aiguille trocart située dans la lumière de la canule du cathéter, une fente peut subsister dans le bouchon à l'endroit du passage de l'aiguille se traduisant par un manque d'étanchéité lors de la perfusion d'un liquide par exemple à partir de la branche latérale.

Toutefois, le remplacement du caoutchouc siliconé par le latex ne pourrait pas constituer une solution acceptable au problème évoqué précédemment. En effet, des essais d'orientation effectués dans le cadre de la présente invention ont montré que l'utilisation d'un bouchon en latex pour l'obturation de l'embase creuse d'un cathéter s'accompagne également d'un manque d'étanchéité aux liquides lors de la mise en place et de l'usage de ce cathéter.

Par ailleurs, l'emploi du latex à des fins médicales devient de plus en plus controversé étant donné le pouvoir allergisant manifesté chez un nombre croissant de patients lors d'un contact avec ce produit.

La présente invention a pour but de proposer un bouchon d'obturation de l'embase creuse d'un cathéter, en particulier un cathéter en Y, qui permet de pallier les inconvénients de l'état de la technique en ce qu'il est apte à assurer l'étanchéité nécessaire de cette embase lors du retrait de l'aiguille hors de la canule et durant la totalité de la période de mise en place du cathéter chez un patient.

Pour atteindre ce but, le bouchon, du type indiqué précédemment, est caractérisé en ce que la composition élastomérique comprend un polyisoprène synthétique soumis à vulcanisation et additionné de charges.

La composition élastomérique en question correspond à un caoutchouc synthétique d'isoprène à savoir un caoutchouc dépourvu de substances naturelles. En particulier, ce caoutchouc ne contient pas ou ne provient pas de latex naturel mais comprend, pour l'essentiel, un élastomère constitué de polyisoprène synthétique soumis à vulcanisation et additionné de charges. Il est d'autre part exempt de protéines, de silicone ou dérivés siliconés ou encore de 2-mercaptobenzothiazole.

Selon une autre caractéristique de l'invention, le caoutchouc synthétique d'isoprène en question peut être sélectionné parmi les compositions élastomériques de formulation générale I suivante comprenant :
- un élastomère, à savoir un polyisoprène synthétique,
- un système de renforcement comprenant, en particulier, des minéraux inertes, de préférence des oxydes métalliques,
- un système de vulcanisation, en particulier le soufre,
et répondant aux caractéristiques ci-dessous :

| | |
|---|---|
| Dureté au duromètre^{(a)} | 20 à 40 |
| (Shore A) | |
| Compression^{(b)} | 45 à 65 |
| (% de la déflexion initiale) | |
| Perméabilité à la vapeur d'eau^{(c)} | 5 à 8 |
| (g/m²/jour) | |
| Perméabilité à l'oxygène^{(d)} | 3000 à 4000 |
| (cc/m²/jour) | |

Plus particulièrement, les compositions élastomériques utiles dans le cadre de l'invention, peuvent être choisies parmi les compositions de formulation générale II ci-dessous comprenant :
- un élastomère, à savoir un polyisoprène synthétique,
- un système de renforcement comprenant, en particulier des minéraux inertes, de préférence des oxydes métalliques,
- un système de vulcanisation, en particulier le soufre,
et répondant aux caractéristiques ci-après :

| | |
|---|---|
| Dureté au duromètre^{(a)} | 25 à 35 |
| (Shore A) | |
| Compression^{(b)} | 50 à 60 |
| (% de la déflexion initiale) | |
| Perméabilité à la vapeur d'eau^{(c)} | 6 à 7 |
| (g/m²/jour) | |
| Perméabilité à l'oxygène^{(d)} | 3400 à 3600 |
| (cc/m²/jour) | |

(a) le duromètre mesure la capacité de la composition élastomérique à résister à l'indentation dans des conditions qui ne perforent pas le caoutchouc. La méthode utilisée est celle de l' « American Society for Testing and Materials » à savoir la méthode ASTM Partie 9 D-2240.
(b) le test de compression mesure la capacité de la composition élastomérique à retrouver ses dimensions à l'origine après élimination d'une force de déformation. Cette capacité de déformation rémanente après compression est définie comme équivalant au pourcentage de l'épaisseur originale d'un échantillon élastomérique à tester après qu'une force ait été appliquée pendant une durée déterminée selon la méthode ASTM Partie 9 D-395.
(c) le test de perméabilité à la vapeur mesure la capacité de la composition élastomérique à transmettre de l'humidité.
(d) le test de perméabilité à l'oxygène mesure la capacité de la composition élastomérique à transmettre de l'oxygène.

Parmi l'ensemble des compositions élastomériques utilisables définies précédemment pour l'élaboration du bouchon selon l'invention, la composition élastomérique répondant aux caractéristiques suivantes, ci-après dénommée « Composition élastomérique A », s'est révélée Particulièrement avantageuse :

| | Résultats | Méthode utilisée |
|---|---|---|
| Dureté au duromètre | 30 | ASTM Partie 9 |
| (Shore A) | | D-2240 |
| Compression | 54,1 | ASTM Partie 9 |
| | | D-395 |
| Perméabilité à la vapeur d'eau | 6,6 | |
| (g/m²/jour) | | |
| Perméabilité à l'oxygène | 3490,2 | |
| (cc/m²/jour) | | |

En outre, cette composition élastomérique préférée présente également les caractéristiques suivantes :

| | |
|---|---|
| Cendres | 6,5 +/- 1,0 |
| (%) | |
| Poids spécifique | 0,95 +/- 0,04 |
| Spectre U.V. | conforme (Figure 1 ci-annexée) |
| Spectre I.R. | conforme (Figure 2 ci-annexée) |

Le bouchon élastomérique selon l'invention étant destiné à un usage en relation avec des produits pharmaceutiques injectables devra satisfaire à des critères et standards particuliers définis selon certaines pharmacopées notamment selon la section 381 de la « United States Pharmacopeia (USP) ». Cette section décrit en fait des tests de changement de pH, de turbidité, d'agents réducteurs, de matières extractibles et de métaux lourds destinés à définir des caractéristiques physico-chimiques obtenues après extraction dans le cas particulier de systèmes de bouchage ou de fermeture pour produits injectables.

Pour certains de ces tests, on a mis en oeuvre la méthode USP 381, le solvant d'extraction étant le solvant A selon cette méthode à savoir l'eau purifiée.

Des caractéristiques de l'extrait ainsi obtenu au départ d'un bouchon réalisé avec la « Composition élastomérique A » sont reprises ci-dessous :

| Test | Résultats | Méthodes |
|---|---|---|
| Changement de pH (unité de pH) | 14 | USP 381 |
| Turbidité (UNT)* | 1,1 | USP 381 |
| Agents réducteurs (ml d'iode 0,01 N) | 0,2 | USP 381 |
| Matières extractibles totales (mg/100 ml) | 0 | USP 381 |

| Métaux lourds | | |
|---|---|---|
| • Plomb (ppm) | 0,1 | |
| • Zinc (ppm) | 0,4 | |
| Spectre U.V. | Figure 3 ci-annexée | |

| | | |
|---|---|---|
| * unité néphélométrique de turbidité | | |

Des tests complémentaires de toxicité systémique et d'irritation intra cutanée pratiqués avec la « Composition élastomérique A » en vue de déterminer la réaction de tissus normaux d'animaux et la réaction d'animaux vivants à la présence d'extraits de ce matériau à tester ont montré que la composition en question est conforme aux exigences de ces tests.

L'ensemble des caractéristiques mises ainsi en évidence permet de conclure, par conséquent, que la « Composition élastomérique A » remplit les conditions nécessaires, notamment d'innocuité, pour un usage à titre pharmaceutique.

En conséquence, la composition en question peut être valablement envisagée pour la réalisation d'éléments constitutifs de cathéters, c'est-à-dire de dispositifs intraveineux comprenant une canule solidarisée à l'extrémité distale d'une embase creuse dont l'extrémité proximale comporte des moyens de verrouillage à une chambre extérieure de visualisation.

Cette embase, préférentiellement transparente et sensiblement cylindrique, est habituellement et préférentiellement munie d'une branche latérale ou conduit latéral.

Cette branche, dont l'une des extrémités débouche dans l'évidement de l'embase en question, forme un angle avec l'axe longitudinal de celle-ci, conférant à l'ensemble la forme d'un Y.

Pour cette raison, un cathéter comprenant une telle embase creuse pourvue d'une branche latérale prendra la dénomination de « cathéter en Y » ou cathéter uniponction.

Ce cathéter en Y peut résulter de l'adjonction latérale d'une branche supplémentaire au niveau de l'embase creuse d'un cathéter droit ou au niveau d'une embase creuse formant un angle avec la canule. Dans l'un comme dans l'autre cas, la jonction de la branche latérale avec l'embase se situe plutôt à proximité de l'extrémité distale de celle-ci.

Cette branche latérale se présente, pour l'essentiel, sous la forme d'un conduit, avantageusement tubulaire, pourvu d'une embase de protection située à l'extrémité de jonction avec l'embase creuse. L'extrémité libre de ce conduit, quant à elle, peut être munie d'accessoires généralement utilisés pour l'administration de liquides ou de médicaments tels que par exemple une valve pour une telle administration au moyen d'une seringue avec ou sans aiguille.

Un cathéter en Y du type ainsi décrit est habituellement utilisé en anesthésie, pédiatrie, oncologie, pour le contrôle de l'analgésie du patient, pour le traitement de douleurs post-opératoires ou autres.

L'embase creuse du cathéter en question comporte en outre, à son extrémité proximale, des moyens de verrouillage aptes à coopérer avec des moyens de verrouillage complémentaires au niveau d'un support d'aiguille comprenant une chambre de visualisation solidarisée à une aiguille logée dans la canule. Ces différents moyens sont identiques ou analogues, par exemple, à ceux qui sont décrits dans la demande de brevet EP 0 169 704.

Selon une mise en oeuvre préférée, les moyens de verrouillage au niveau de l'embase creuse du cathéter comprennent des ergots qui émergent de la face externe de sa paroi ainsi qu'un orifice d'extrémité apte à recevoir, pour son obturation, une portion complémentaire du support d'aiguille.

Ainsi, la canule du cathéter en question comprend, à l'intérieur de sa lumière, une aiguille creuse formant trocart dont l'extrémité distale est munie d'un biseau reliant deux points diamétralement opposés de sa paroi extérieure et formant un angle avec son axe longitudinal.

Cette aiguille trocart, habituellement en acier inoxydable ayant subi un traitement siliconé, comporte une extrémité proximale solidarisée à l'extrémité distale d'une embase transparente, correspondant à la chambre de visualisation du support d'aiguille, embase habituellement en matériau synthétique tel que le polyméthylpentène et dont l'extrémité proximale est munie d'un bouchon amovible. Cette chambre est destinée à visualiser le reflux sanguin lors de la ponction.

Par ailleurs, ce support d'aiguille présente des moyens de verrouillage à l'embase creuse du cathéter comprenant une portion distale, avantageusement creuse, de forme complémentaire à celle de l'orifice d'extrémité de l'embase creuse ainsi que deux bras opposés, articulés à la face externe de la paroi de la chambre de visualisation et aptes à s'encliqueter sous les ergots au niveau de l'embase creuse.

Avantageusement, la portion distale de chacun de ces bras présente un rebord dirigé vers la chambre de visualisation, ceux-ci étant destinés à faciliter l'encliquetage en question.

Des tests comparatifs complémentaires ont été réalisés, dans le cadre de la présente invention, en vue de mettre en évidence les qualités d'étanchéité d'un bouchon d'obturation d'embase creuse de cathéter, réalisé à partir d'une composition élastomérique selon l'invention en particulier la « Composition élastomérique A ».

Dans ces tests, on a utilisé 5 lots chacun de 9 cathéters en Y conservés au four à 40°C pendant 4 mois et dont les dimensions de la canule variaient de 23 à 16 gauges c'est-à-dire de 0,573 mm à 1,201 mm, l'embase de chaque cathéter étant munie d'un bouchon réalisé à partir de la « Composition élastomérique A ».

Ces tests ont été pratiqués comparativement à des lots contrôles constitués de cathéters en Y identiques aux précédents mais dont le bouchon d'obturation de l'embase était en latex naturel.

Le test a consisté à introduire, durant 2 min, de l'eau sous pression de 0,5 bar dans la chambre de raccord ou embase du cathéter. Cette introduction s'est effectuée par la branche latérale de ce dernier tout en ôtant l'aiguille trocart de la canule par traction au travers du bouchon en question.

Au moment du retrait complet de l'aiguille, des éclaboussures peuvent se produire et des fuites à l'endroit du passage de cette aiguille peuvent subsister traduisant un manque d'étanchéité des bouchons. On a alors noté le nombre de cathéters présentant des éclaboussures et/ou des fuites persistantes au niveau du bouchon. Les résultats obtenus sont listés ci-dessous .

**A. Cathéters avec bouchons en latex**

| | Nombre de cathéters présentant : | |
|---|---|---|
| Dimensions de la lumière de la canule (gauge) | des éclaboussures sans fuite lors du retrait de l'aiguille | des fuites à travers le bouchon |
| 23 | 3 | 0 |
| 21 | 5 | 0 |
| 19 | 1 | 4 |
| 18 | 8 | 5 |
| 16 | 0 | 0 |

**B. Cathéters avec bouchons réalisés à partir de la « Composition élastomérique A »**

| | Nombre de cathéters présentant : | |
|---|---|---|
| Dimensions de la lumière de la canule (gauge) | des éclaboussures sans fuite lors du retrait de l'aiguille | des fuites à travers le bouchon |
| 23 | 1 | 0 |
| 21 | 3 | 0 |
| 19 | 1 | 0 |
| 18 | 0 | 0 |
| 16 | 0 | 0 |

Les résultats enregistrés montrent que lorsque les bouchons d'obturation montés sur l'embase de cathéters sont réalisés à partir de la « Composition élastomérique A » :
- seulement 20 % de ces bouchons ont produit des éclaboussures contre 37,7 % des bouchons en latex naturel,
- aucune fuite ne se présente au niveau de ces bouchons alors que 36 % des bouchons en latex présentent des fuites.

En conclusion, 62 % des bouchons réalisés à partir de la « Composition élastomérique A » ont montré une étanchéité totale contre seulement 36 % des bouchons en latex naturel, ce qui démontre une nette supériorité des bouchons comprenant un polyisoprène synthétique.

L'invention sera mieux comprise et d'autres buts, caractéristiques et avantages de celle-ci apparaîtront plus clairement au cours de la description explicative qui va suivre faite en référence aux dessins schématiques donnés uniquement à titre d'exemples illustrant des modes de réalisation de l'invention et dans lesquels :

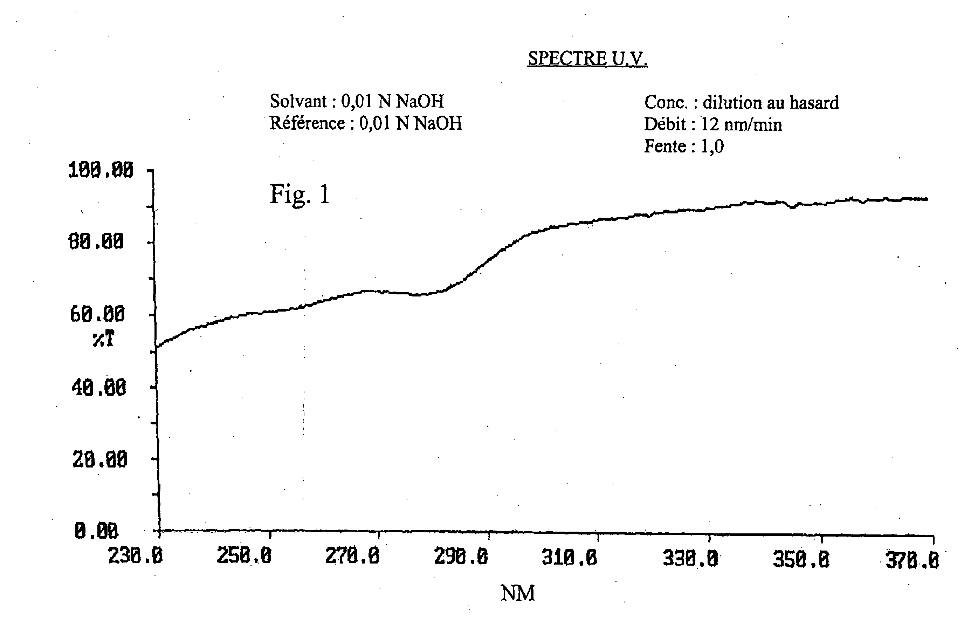

- la figure 1 montre un spectre U.V. de la « Composition élastomérique A »,

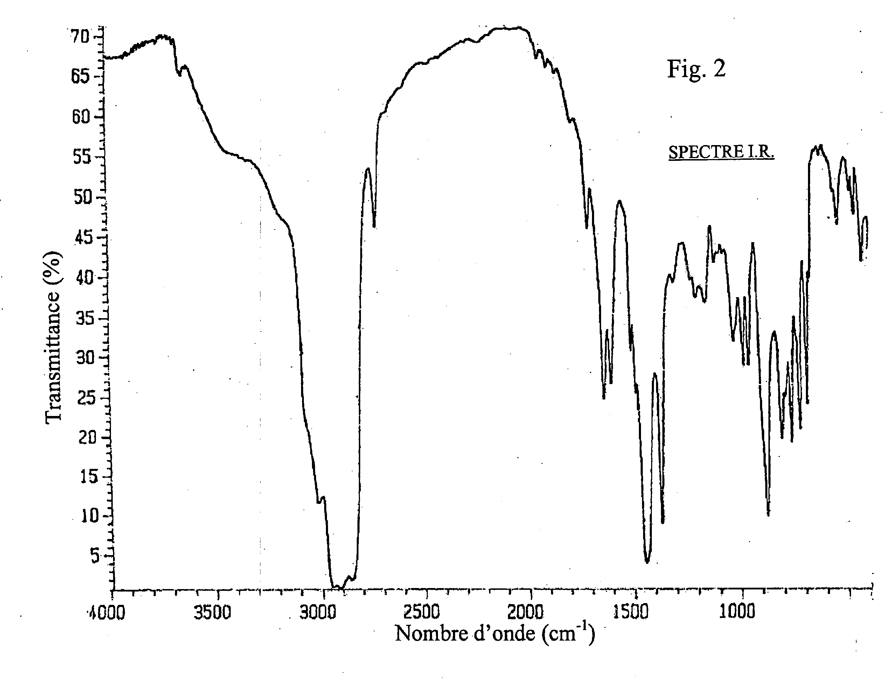

- la figure 2 montre un spectre I.R. de la « Composition élastomérique A »,

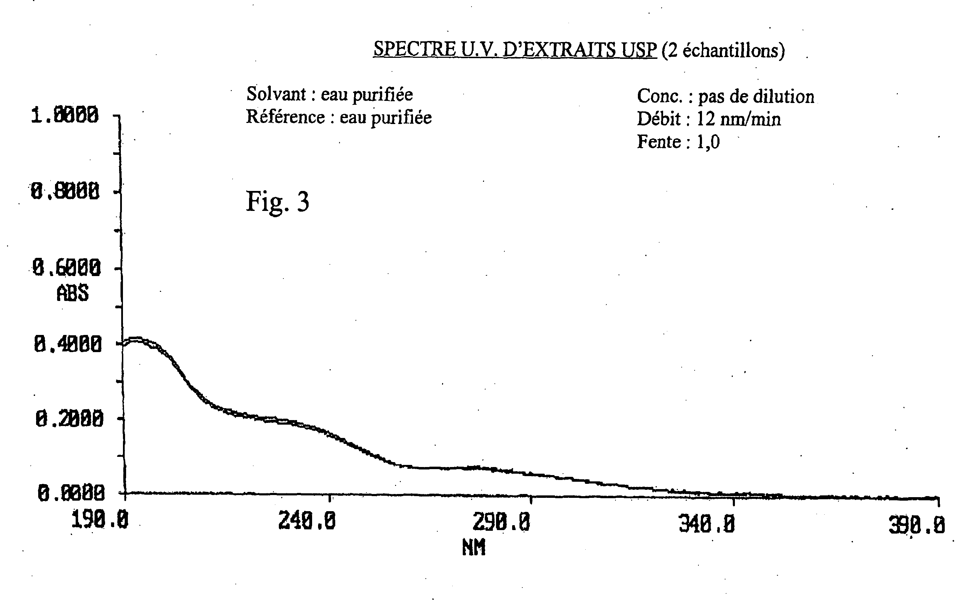

- la figure 3 montre un spectre U.V. d'extraits obtenus au départ d'un bouchon réalisé avec la « Composition élastomérique A »,
- la figure 4 montre une vue en coupe frontale d'un cathéter en Y muni du bouchon conforme à l'invention,
- la figure 5 montre une vue frontale d'un support d'aiguille adaptable sur le cathéter à la figure 4.
Tel que représenté à la figure 4, la canule 1 formée d'un tube cylindrique en éthylène-propylène fluoré (TEFLON®) est solidaire d'une embase 2, creuse et transparente, réalisée en chlorure de polyvinyle (PVC) et formant chambre de raccord. Cette chambre est partiellement solidarisée à la canule.

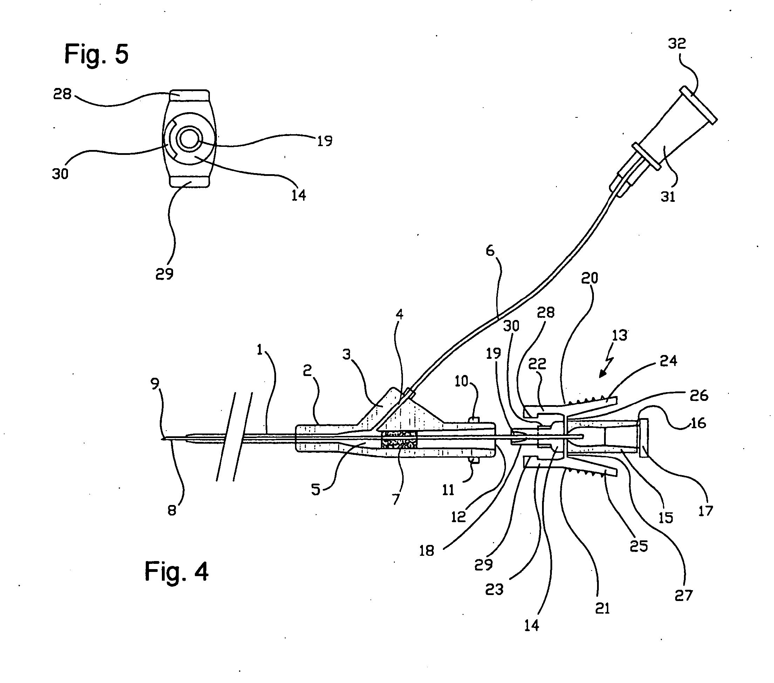

Comme le montre également la figure 4, l'embase est munie d'un embranchement 3 latéral traversé par un conduit 4 débouchant dans la chambre 5 de l'embase, ce conduit se prolongeant en un tube 6, fin et souple, pour une administration continue ou discontinue de médicaments ou de liquides. On remarque également que la chambre 5 de l'embase est pourvue d'un bouchon 7. Ce bouchon, avantageusement en caoutchouc synthétique d'isoprène et préférentiellement de formulation répondant à la « Composition élastomérique A », comprime légèrement et de manière étanche la paroi de la chambre 5. Il est en outre juxtaposé à la jonction de la portion distale de la chambre 5 avec le conduit 3 latéral en sorte qu'un liquide, s'écoulant par le conduit tubulaire 6, peut franchir librement cette chambre en direction de la canule.

En se référant à nouveau à la figure 4, on observe également une aiguille 8 creuse, formant trocart, insérée dans la lumière de la canule 1, l'extrémité distale de cette aiguille, en acier inoxydable siliconé, comportant un biseau 9. On remarque, en outre, que l'extrémité proximale de la chambre 5 de raccord, extrémité avantageusement cylindrique, présente deux ergots 10 et 11 diamétralement opposés et un orifice 12. Cet orifice est apte à coopérer avec un support d'aiguille 13 amovible destiné à obturer la portion proximale de cette chambre 5 et à empêcher le retrait de l'aiguille hors de la canule. Ce support d'aiguille comprend un corps 14 circulaire solidarisé à une embase 15, transparente et creuse, formant chambre de visualisation. Celle-ci présente, à son extrémité proximale, un orifice 16 obturable par un bouchon 17 et à son extrémité distale une portion 18, apte à s'engager dans l'orifice 12 pour son obturation. Cette portion 18 est creuse en ce qu'elle comporte un orifice s'ouvrant sur l'évidement 19 en forme de tulipe lequel assure le passage de l'extrémité proximale de l'aiguille 8 fixée à demeure dans le corps 14. Ce corps est, par ailleurs, solidarisé à cette extrémité de l'aiguille qui débouche dans la chambre de visualisation 15.

En se référant à nouveau à la figure 4, on distingue une paire de bras 20 et 21 montés en opposition sur le corps 14. Ces bras sont formés chacun d'une portion distale, respectivement 22 et 23, dirigée vers la portion creuse 18 et d'une portion proximale, respectivement 24 et 25, dirigée vers l'orifice 16 tandis que la face extérieure de cette portion proximale comporte une pluralité de stries ou de saillies de manière à permettre la préhension aisée avec les doigts. En outre, ces bras sont reliés chacun au corps 14 par l'intermédiaire de pontets, respectivement 26 et 27, l'ensemble venant de moulage à partir d'une matière plastique, par exemple le polypropylène. Ces pontets, étant donné leur souplesse, sont capables de provoquer le pivotement des bras 20 et 21 lors d'une pression exercée sur les portions proximales 24 et 25, par exemple par les doigts d'un utilisateur, en sorte que ces portions se rapprochent de la chambre 15 tandis que les portions distales 22 et 23 s'en éloignent.

Au surplus, on observe que les portions 22 et 23 des bras 20 et 21 sont munies de rebords respectivement 28 et 29. Ceux-ci sont dirigés vers la portion creuse 18 tandis que le corps 14 laisse apparaître une saillie 30, visible également à la figure 5, épousant partiellement le pourtour circulaire de celui-ci. La largeur de cette saillie, positionnée entre les bras 20 et 21 est, par ailleurs, sensiblement égale à la distance séparant deux extrémités latérales rapprochées des ergots 10 et 11. Ainsi, lors d'une pénétration de la portion creuse 18 dans l'embase 2, la saillie 30 s'engage entre les ergots 10 et 11 jusqu'à amener le corps 14 en butée contre l'embase 2 du cathéter et à empêcher la rotation de cette portion 18 dans l'embase 2. Ce faisant, les rebords 28 et 29 viennent s'encliqueter sous les ergots 10 et 11 respectivement en sorte qu'une traction axiale exercée sur la chambre de visualisation empêche le retrait de l'aiguille logée dans la canule. Toutefois, ce retrait peut être obtenu en exerçant une légère pression sur les portions proximales 24 et 25 des bras 20 et 21 respectivement et ce, en direction de la chambre 15, ce qui provoque le désengagement des rebords 28 et 29 et la possibilité de retrait de l'aiguille par traction axiale sur le support 13.

En se référant à nouveau à la figure 4, on remarque, à l'extrémité libre du conduit tubulaire 6, une valve 31 raccordée à ce tube par l'intermédiaire d'un système « Luer lock ». Cette valve comporte un site pour l'injection par exemple de médicaments en bolus au moyen d'une seringue sans aiguille, le site d'injection étant protégé par un capuchon 32 articulé au niveau de la paroi de cette valve.

Selon un autre mode de réalisation, cette valve peut être dépourvue de capuchon articulé mais comporter une coupelle de protection centrée sur un site d'injection au moyen d'une seringue avec aiguille.

Le bouchon selon l'invention pour l'obturation de l'embase creuse d'un cathéter présente les avantages du bouchon en latex naturel tout en étant dépourvu de ses inconvénients.

Ainsi le bouchon selon l'invention comme le bouchon en latex est biocompatible et moulable par injection.

D'autre part, l'un et l'autre gardent leurs propriétés à la stérilisation, ce qui les rend stérilisables. Toutefois, le bouchon selon l'invention, au contraire du bouchon en latex, n'est pas allergisant, présente une meilleure étanchéité et est doué d'une résistance supérieure au vieillissement puisqu'il maintient ses propriétés durant un laps de temps plus prolongé.

## Revendications

1. Bouchon, réalisé en une composition élastomérique, pour l'obturation d'une embase creuse de cathéter reliée à une canule, en particulier une embase de cathéter conformé en Y, **caractérisé en ce que** la composition élastomérique correspond à un caoutchouc synthétique d'isoprène comprenant un élastomère constitué de polyisoprène synthétique soumis à vulcanisation et additionné de charges.

2. Bouchon selon la revendication 1, **caractérisé en ce que** la composition élastomérique est sélectionnée parmi les compositions élastomériques comprenant :
• un élastomère à savoir le polyisoprène synthétique,
• un système de renforcement comprenant des minéraux inertes,
• un système de vulcanisation comprenant du soufre,
et répond aux caractéristiques ci-dessous :
| | |
|---|---|
| Dureté au duromètre | 20 à 40 |
| (Shore A) | |
| Compression | 45 à 65 |
| (% de la déflexion initiale) | |
| Perméabilité à la vapeur d'eau | 5 à 8 |
| (g/m²/jour) | |
| Perméabilité à l'oxygène | 3000 à 4000 |
| (cm²/jour) | |

3. Bouchon selon la revendication 2, **caractérisé en ce que** la composition élastomérique répond aux caractéristiques suivantes :
| | |
|---|---|
| Dureté au duromètre | 25 à 35 |
| (Shore A) | |
| | |
|---|---|
| Compression | 50 à 60 |
| (% de la déflexion initiale) | |
| Perméabilité à la vapeur d'eau | 6 à 7 |
| (g/m²/jour) | |
| Perméabilité à l'oxygène | 3400 à 3600 |
| (cc/m²/jour) | |

4. Bouchon selon la revendication 3, **caractérisé en ce que** la composition élastomérique répond aux caractéristiques suivantes :
| | |
|---|---|
| Dureté au duromètre | 30 |
| (Shore A) | |
| Compression | 54,1 |
| (% de la déflexion initiale) | |
| Perméabilité à la vapeur d'eau | 6,6 |
| (g/m²/jour) | |
| Perméabilité à l'oxygène | 3490,2 |
| (cc/m²/jour) | |
| Cendres | 6,5 +/- 1,0 |
| (%) | |
| Poids spécifique | 0,95 +/- 0,04 |

## Claims

1. A plug made from an elastomeric composition for the obturation of a hollow base of a catheter connected to a cannula, in particular a base of a Y-configured catheter, **characterised in that** the elastomeric composition is a synthetic isoprene rubber comprising an elastomer consisting of a synthetic polyisoprene with an addition of fillers and subjected to vulcanisation.

2. A plug according to Claim 1, **characterised by** the fact that the elastomeric composition is selected from among elastomeric compositions comprising
- an elastomer, namely, synthetic polyisoprene
- a system of reinforcements comprising inert minerals
- a system of vulcanisation comprising sulphur and possessing the following characteristics
| | |
|---|---|
| Durometer hardness (Shore Hardness A) | 20 to 40 |
| Compression (% of initial deflection) | 45 to 65 |
| Water vapour permeability (g/m²/day) | 5 to 8 |
| Oxygen permeability (cm²/day) | 3000 to 4000 |

3. A plug according to Claim 2, **characterised by** the fact that the elastomeric composition possesses the following characteristics
| | |
|---|---|
| Durometer hardness (Shore Hardness A) | 25 to 35 |
| | |
|---|---|
| Compression (% of initial deflection) | 50 to 60 |
| Water vapour permeability (g/m²/day) | 6 to 7 |
| Oxygen permeability (cm³/m²/day | 3400 to 3600 |

4. A plug according to Claim 3, **characterised by** the fact that the elastomeric composition possesses the following characteristics
| | |
|---|---|
| Durometer hardness (Shore Hardness A) | 30 |
| Compression (% of initial deflection) | 54.1 |
| Water vapour permeability (g/m²/day) | 6.6 |
| Oxygen permeability | 3490.2 |
| (cm³/m²/day) | |
| Ash (%) | 6.5 ± 1.0 |
| Specific gravity | 0.95 ± 0.04 |

## Patentansprüche

1. Stopfen, hergestellt aus einer elastomerischen Zusammensetzung zum Abdichten eines hohlen Kathetersockels, der mit einer Kanüle verbunden ist, insbesondere eins Kathetersockels in Form eines Y, **dadurch gekennzeichnet, dass** die elastomerische Zusammensetzung einem synthetischen Isoprengummi entspricht, umfassend ein Elastomer aus synthetischem Polyisopren, das der Vulkanisation unterworfen ist, und dem Ladungen hinzugefügt sind.

2. Stopfen nach Anspruch 1, **dadurch gekennzeichnet, dass** die elastomerische Zusammensetzung aus den elastomerischen Zusammensetzungen ausgewählt wird, die Folgendes umfassen:
- ein Elastomer, d.h. das synthetische Polyisopren,
- ein Verstärkungssystem, umfassend inerte Mineralien,
- ein Vulkanisierungssystem, umfassend Schwefel,
und die unten stehenden Eigenschaften aufweist:
| | |
|---|---|
| Härte im Härtemesser (Shore A) | 20 bis 40 |
| Verdichtung (% der ursprünglichen Ablenkung) | 45 bis 65 |
| Durchlässigkeit für Wasserdampf (g/m²/Tag) | 5 bis 8 |
| Durchlässigkeit für Sauerstoff (cm²/Tag) | 3000 bis 4000 |

3. Stopfen nach Anspruch 2, **dadurch gekennzeichnet, dass** die elastomerische Zusammensetzung die folgenden Eigenschaften umfasst:
| | |
|---|---|
| Härte im Härtemesser (Shore A) | 25 bis 35 |
| | |
|---|---|
| Verdichtung (% der ursprünglichen Ablenkung) | 50 bis 60 |
| Durchlässigkeit für Wasserdampf (g/m²/Tag) | 6 bis 7 |
| Durchlässigkeit für Sauerstoff (cc/m²/Tag) | 3400 bis 3600 |

4. Stopfen nach Anspruch 3, **dadurch gekennzeichnet, dass** die elastomerische Zusammensetzung den folgenden Eigenschaften entspricht:
| | |
|---|---|
| Härte im Härtemesser (Shore A) | 30 |
| Verdichtung (% der ursprünglichen Ablenkung) | 54,1 |
| Durchlässigkeit für Wasserdampf (g/m² /Tag) | 6,6 |
| Durchlässigkeit für Sauerstoff (cc/cm²/Tag) | 3490,2 |
| Aschen (%) | 6,5 +/- 1,0 |
| Dichte | 0,95 +/- 0,04 |
